# NEUE EUROPÄISCHE PATENTSCHRIFT

(11) **EP 0 791 350 B2**
(45) Veröffentlichungstag und Bekanntmachung des Hinweises auf die Entscheidung über den Einspruch: **05.04.2006**
(45) Hinweis auf die Patenterteilung: 08.01.2003
(21) Anmeldenummer: 97102162.1
(22) Anmeldetag: 12.02.1997
(51) Int. Cl.: A61Q 5/04, A61K 8/44

(54) **Mittel zur dauerhaften Verformung von menschlichen Haaren**
Composition for permanent waving of human hair
Composition pour l'ondulation permanente des cheveux humains

(30) Priorität: 22.02.1996 DE 19606572
(43) Veröffentlichungstag der Anmeldung: 27.08.1997
(73) Patentinhaber: KPSS-Kao Professional Salon Services GmbH, 64297 Darmstadt (DE)
(72) Erfinder: Nöcker, Bernd, Dr., 64372 Ober-Ramstadt (DE); Rose, Burkhard, 64297 Darmstadt (DE)

(56) Entgegenhaltungen:
- EP-A- 0 362 663
- EP-A- 0 614 657
- EP-A- 0 727 202
- DE-A- 948 501
- DE-A- 3 138 142
- JP-A- 5 486 635
- US-A- 4 459 284
- US-A- 5 223 252
- US-A- 5 332 570

## Beschreibung

Die vorliegende Erfindung betrifft ein Dauerwellmittel mit guter Wellwirksamkeit, das jedoch gleichwohl eine haarschonende Behandlung gewährleistet.

Die Dauerwellung erfolgt bekanntlich in zwei Behandlungsschritten, der reduktiven Spaltung der Cystin-Disulfidbrücken des Haares durch Einwirkung eines Reduktionsmittels, und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch die Thioglykolsäure, auch in Form ihrer Salze, insbesondere des Ammoniumsalzes, obwohl zahlreiche andere Thioverbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen 8 und 10, insbesondere 8,5 und 9,5, eingesetzt, was bei wiederholter, zeitlich nahe zusammenliegender Anwendungen zu Haarschädigungen führen kann.

Es wurde daher auch schon vorgeschlagen, Dauerwellen im schwach sauren, neutralen bzw. schwach alkalischen pH-Bereich zwischen etwa 6,5 und 8,5 anzuwenden.

Das hierfür meist verwendete Reduktionsmittel Glycerinmonothioglykolat wird jedoch aufgrund seiner sensibilisierenden und allergisierenden Nebenwirkung heute kaum mehr eingesetzt.

Eine Lösung dieses Problems ist aus der DE-C 43 04 828 bekannt: Der Einsatz eines Mittels, das Thioglykolsäure bzw. ein Salz derselben enthält und unmittelbar vor der Anwendung durch Vermischen von zwei bis dahin getrennt gehaltenen Zusammensetzungen hergestellt wird, wobei die eine Zusammensetzung (A) mindestens ein Aminosäurehydrochlorid und mindestens ein Polyol bzw. dessen Methyl- und Ethylether auf wäßriger Grundlage enthält, und einen pH-Wert zwischen 4,5 und 6,5, vorzugsweise zwischen 5 und 6, aufweist, und eine Zusammensetzung (B), die als Alkalisierungsmittel Ammoniumhydrogencarbonat, Ammoniumcarbonat und/oder Ammoniumcarbamat enthält und einen pH-Wert zwischen etwa 8 und 9,5 aufweist, und das beim Vermischen beider Zusammensetzungen erhaltene, gebrauchsfertige Mittel (AB) einen pH-Wert zwischen 7 und 8 besitzt.

Als Aminosäurehydrochlorid wird in dieser Zusammensetzung vorwiegend Cysteinhydrochlorid eingesetzt.

Es wurde jedoch überraschend festgestellt, daß Cysteinhydrochlorid, das als Reduktionsmittel wirkt, den Nachteil aufweist, im dauergewellten Haar nach der Dauerwellung und Fixierung freie Disulfide zurückzulassen, was nicht nur die Haltbarkeit der Dauerwelle beeinträchtigen, sondern auch zu Geruchsrückständen im Haar führen kann.

Die vorliegende Erfindung hat sich die Aufgabe gestellt, diese Nachteile zu beheben.

Die Lösung dieser Aufgabe besteht nun darin, das in Kombination mit reduzierenden Thioverbindungen in Mitteln zur dauerhaften Verformung von menschlichen Haaren eingesetzte Cysteinhydrochlorid teilweise durch mindestens eine weitere Aminosäure ausgewählt aus Glycin, Citrullin, Histidin und Asparaginsäure bzw. wasserlösliche Salze zu ersetzen, wobei das Gewichtsverhältnis von Cysteinhydrochlorid zu weiterer Aminosäure bzw. deren Salz zwischen 1 : 20 und 2 : 1, jeweils berechnet auf den Gehalt an freier Aminosäure, liegt.

Ein besonders bevorzugtes Gewichtsverhältnis von Cystein zu weiterer Aminosäure liegt bei 1 : 1 und 1 : 10, insbesondere 1 : 2 bis 1 : 8.

Durch diese teilweise Substitution wird überraschenderweise die Wellwirksamkeit des Mittels nicht beeinträchtigt, was nach den Ausführungen in der DE-C 43 04 828 und insbesondere auch der DE-A 44 28 574 zu erwarten gewesen wäre.

Die Gesamtmenge des Gemisches Cysteinhydrochlorid/Aminosäure(n) liegt zwischen etwa 0,25 und etwa 10, vorzugsweise 0,5 bis 7,5, insbesondere 1 bis 5 Gew.-% der reduzierenden Dauerwellzusammensetzung.

Der Einsatz von Aminosäuren per se in Dauerwelimitteln ist, neben den bereits erwähnten DE-C 43 04 828 und der DE-A 44 28 574, auch aus der US-A 4 459 284 bekannt, jedoch läßt sich diesem Stand der Technik keinerlei Hinweis auf die erfindungsgemäßen Zusammensetzungen entnehmen.

Die erfindungsgemäßen Dauerwellmittel enthalten mindestens eine reduzierende Thioverbindung. Bevorzugt sind Thioglykolsäure und Thiomilchsäure sowie deren Salze, insbesondere die Ammonium- und Ethanolaminsalze.

Weitere einsetzbare Thioverbindungen sind N-Acetylcystein, Thioglycerin, Ethandiolmonothioglykolat, 1,2-Propylenglykolmonothioglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Ethandiolmonothiolactat, 1,2-Propandiol- und 1,3-Propandiolmonothiolactat und deren Isomerengemische, 1,3-Butandiol- und 1,4-Butandiolmonothiolactat und deren Isomerengemische, Polyethylenglykol- wie Di-, Tri- und Tetraethylenglykolmonothioglykolate und -monothiolactate, Polypropylenglykol- wie Di-, Tri- und Tetrapropylenglykolmonothiolactate und -monothioglykolate, Glycerinmonothiolactate und weitere Thiosäuren und deren Ester sowie Gemische derselben.

Der Gesamtgehalt an Reduktionsmitteln in den erfindungsgemäßen Zusammensetzungen beträgt üblicherweise etwa 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel im Rahmen der Erfindung sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Die erfindungsgemäß zum Einsatz kommenden Dauerwellmittel enthalten vorzugsweise auch Tenside. Deren Anteil liegt bei etwa 0,1 bis etwa 10, insbesondere etwa 1 bis etwa 5 Gew.-%, der das Reduktionsmittel enthaltenden Zusammensetzung.

Sowohl bei den in den Reduktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind besonders die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C8-C18-Fettalkoholpolyglykolether, Fettsäurepolyglykolester, Fettsäurealkanolamide, Aminoxide und vor allem C₈-C₁₈-Alkylpolyglukoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer wünschenswerter Bestandteil der erfindungsgemäß verwendeten Reduktionsmittel-Zusammensetzungen ist ein C₃-C₆-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Wellmittels.

Die erfindungsgemäß eingesetzten Mittel können selbstverständlich alle in Dauerwellmitteln üblichen Stoffe enthalten, auf deren detaillierte Aufzählung hier verzichtet wird, und als (wäßrige) Lösungen, Emulsionen, Cremes, Schäume etc. vorliegen.

Es kann sich dabei um einphasige Produkte oder um in getrennten Verpackungen untergebrachte Zusammensetzungen handeln, die bei der Anwendung vereinigt werden, wie sie z. B. in der bereits erwähnten DE-C 43 04 828 beschrieben sind.

Zur Vermeidung von Wiederholungen wird hierzu auf den Stand der Technik verwiesen, wie er beispielsweise in "Ullmann's Encyclopedia of Industrial Chemistry", Vol. A12 (1986), S. 588 bis 591, sowie insbesondere in der Monographie von K. Schrader, "Grundlagen und Rezepturen der Kosmetika", 2. Aufläge (1989, Hüthig-Verlag), S. 823 bis 840, sowie in dem Übersichtsartikel von D. Hollenberg et al. in "Seifen-Öle-Fette-Wachse" 117 (1991), S. 81 - 87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit Behandlung durch die üblichen und aus dem Stand der Technik hinreichend bekannten Peroxid- oder Bromat-Zusammensetzungen.

Ebenso kann selbstverständlich eine an sich bekannte Zwischenbehandlung zwischen Reduktions- und Neutralisationsphase erfolgen.

Die folgenden Beispiele dienen der Illustration der Erfindung.

### Beispiel 1

| Zusammensetzung A: | |
|---|---|
| Ammoniumhydrogencarbonat | 4,5 (g) |
| Kationisches Polymer | 1,0 |
| Nichtionischer Lösungsvermittler (Ethylenoxid-Kondensat) | 0,8 |
| 1,2-Propandiol | 1,0 |
| Cocoamidopropylbetain | 1,0 |
| Trübungsmittel, Parfum | q.s. |
| Ammoniak, 25%ig @ | pH 8,6 |
| Wasser @ | 72,0 |

| Zusammensetzung B: | |
|---|---|
| Ammoniumthioglykolat, 70%ig | 18,0 (g) |
| 2-Thiomilchsäure | 1,0 |
| Cysteinhydrochlorid x H₂O | 1,0 |
| Histidinhydrochlorid x H₂O | 2,0 |
| 1,2-Propandiol | 0,5 |
| Ammoniak, 25%ig @ | pH 5,5 |
| Wasser @ | 28,0 |

65 g der Zusammensetzung A und 25 g der Zusammensetzung B wurden getrennt in eine übliche Zweikammerdose verpackt.

Beim Zusammenbringen beider Zusammensetzungen wurde ein Produkt mit einem pH-Wert von 7,3 erhalten, das bei der Anwendung in einer üblichen Dauerwellbehandlung mit anschließender Peroxid-Fixierung eine exzellent ausgebildete, lang haltbare Dauerwellung ohne jedweden Sulfidgeruch erzielte.

### Beispiel 2

| Zusammensetzung A: | |
|---|---|
| Ammoniumhydrogencarbonat | 3,5 (g) |
| Nichtionogener Lösungsvermittler (Ethoxylat) | 0,8 |
| Kationisches Polymer | 1,0 |
| Parfum | 0,3 |
| Ethanol | 0,5 |
| Ethoxydiglykol | 1,0 |
| C₁₂-C₁₄-Alkylpolyglucosid (P.D. = 1,5) | 1,0 |
| Entschäumer, Trübungsmittel | q.s. |
| Ammoniak, 25%ig @ | pH 8,6 |
| Wasser @ | 72,0 |

| Zusammensetzung B: | |
|---|---|
| Ammoniumthioglykolat, 70%ig | 18,0 (g) |
| Cysteinhydrochlorid x H₂O | 0,5 |
| Glycin | 2,0 |
| Ammoniak, 25%ig @ | pH 5,5 |
| Wasser @ | 28,0 |

Die Verpackung und Applikation der Zusammensetzungen A und B erfolgte wie in Beispiel 1 beschrieben.

Die anwendungsfertige Gesamtmischung wies einen pH-Wert von 7,35 auf.

### Beispiel 3

| Zusammensetzung A: | |
|---|---|
| Ammoniumhydrogencarbonat | 5,00 (g) |
| Chlorophyllin | 0,05 |
| Nichtionogener Lösungsvermittler (Ethoxylierungs-Produkt) | 0,80 |
| Ammoniumthiolactat | 1,00 |
| Parfum | 0,50 |
| Kationisches Polymer | 1,50 |
| 1,3-Butandiol | 1,00 |
| Laureth-23 | 1,00 |
| Ammoniak, 25%ig @ | pH 8,6 |
| Wasser @ | 72,00 |

| Zusammensetzung B: | |
|---|---|
| Ammoniumthioglykolat, 70%ig | 18,0 (g) |
| Cysteinhydrochlorid x H₂O | 1,5 |
| Asparaginsäure | 1,5 |
| Ammoniak, 25%ig @ | pH 5,5 |
| Wasser @ | 28,0 |

Die Abpackung und Anwendung erfolgte wie in Beispiel 1 beschrieben; die gebrauchsfertige Mischung wies einen pH-Wert von 7,4 auf.

### Beispiel 4

| Zusammensetzung A: | |
|---|---|
| Ammoniumhydrogencarbonat | 4,5 (g) |
| Kationisches Polymer | 1,0 |
| Nichtionischer Lösungsvermittler (Ethylenoxid-Kondensat) | 0,8 |
| Harnstoff | 1,0 |
| Parfum | 0,3 |
| Trübungsmittel, Entschäumer | q.s. |
| Ammoniak, 25%ig @ | pH 8,6 |
| Wasser @ | 72,0 |

| Zusammensetzung B: | |
|---|---|
| Ammoniumthioglykolat, 70%ig | 18,0 g |
| Cysteinhydrochlorid x H₂O | 1,0 |
| L-Glutaminsäure | 1,0 |
| L-Citrullin | 0,5 |
| Ammoniak, 25%ig @ | pH 5,5 |
| Wasser @ | 28,0 |

Die Abpackung und Anwendung erfolgte, wie in Beispiel 1 beschrieben; die gebrauchsfertige Mischung wies einen pH-Wert von 7,45 auf.

## Patentansprüche

1. Mittel zur dauerhaften Verformung von menschlichen Haaren, enthaltend mindestens eine reduzierende Thioverbindung in einer kosmetisch verträglichen Grundlage, **dadurch gekennzeichnet, daß** es zusätzlich ein Gemisch aus Cysteinhydrochlorid und mindestens einer weiteren Aminosäure ausgewählt aus Glycin, Citrullin, Histidin und Asparaginsäure bzw. deren wasserlöslichen Salzen im Gewichtsverhältnis Cystein zu Aminosäure zwischen 1 : 20 bis 2 : 1 (berechnet auf die freien Aminosäuren) enthält.

2. Mittel nach Anspruch 1, **dadurch gekennzeichnet, daß** das Gewichtsverhältnis von Cystein zu Aminosäure 1 : 1 bis 1 : 10 beträgt.

## Claims

1. Composition for the permanent waving of human hair comprising at least one reducing thio compound in a cosmetically acceptable carrier, additionally containing a mixture of cysteine hydrochloride and at least onf further amino acid, selected from glycine, citrulline, histidine and asparagic acid or the watersoluble salts thereof in a weight ratio of cysteine to amino acid between 1 : 20 to 2 : 1, calculated to free amino acid.

2. Composition according to claim 1, where the weight proportion of cysteine to amino acid is 1 : 1 to 1 : 10.

## Revendications

1. Agent pour la mise en plis permanente de cheveux humains, contenant au moins un composé thio réducteur dans une base compatible avec les cosmétiques, **caractérisé en ce qu'**il contient en plus un mélange de chlorhydrate de cystéine, et d'au moins un acide amine supplémentaire, choisis parmi citrulline, la listidine et l'acide aspartique ou de ses sels hydrosolubles dans un rapport pondéral de cystéine à acide aminé compris entre 1 : 20 à 2 : 1 (calculé par rapport aux acides aminés libres).

2. Agent selon la revendication 1, **caractérisé en ce que**, le rapport de la cystéine à l'acide aminé est de 1 : 1 à 1 : 10.
